Europäisches Patentamt

European Patent Office

Office européen des brevets

(19)

(11) Veröffentlichungsnummer: **0 078 956**
A1

(12) ## EUROPÄISCHE PATENTANMELDUNG

(21) Anmeldenummer: **82109770.6**

(22) Anmeldetag: **22.10.82**

(51) Int. Cl.³: **C 07 C 121/34**
**C 01 D 3/02**

(30) Priorität: **26.10.81 DE 3142375**

(43) Veröffentlichungstag der Anmeldung:
**18.05.83 Patentblatt 83·20**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Mack, Karl Ernst, Dr.**
**Klingenbachstrasse 43**
**D-6200 Wiesbaden(DE)**

(72) Erfinder: **Warning, Klaus, Dr.**
**Kreuzheck 6**
**D-6239 Eppstein/Taunus(DE)**

(54) **Verfahren zur Herstellung von 5-Oxohexannitril.**

(57) Die Erfindung betrifft ein katalytisches Verfahren zur Herstellung von 5-Oxohexannitril aus Aceton und Acrylnitril. Als Katalysator wird ein Alkalifluorid oder ein Alkylammoniumfluorid verwendet, das sich von primären Aminen ableitet.

EP 0 078 956 A1

Croydon Printing Company Ltd

0078956

HOECHST AKTIENGESELLSCHAFT    Dr.MA/AK    HOE 81/F 287

Verfahren zur Herstellung von 5-Oxohexannitril

5-Oxohexannitril ist ein Zwischenprodukt zur Herstellung wichtiger Verbindungen wie $\alpha$-Picolin oder Resorcin.

Es ist bekannt, 5-Oxohexannitril durch Umsetzung von Aceton mit Acrylnitril in Gegenwart eines Katalysators bei erhöhter Temperatur herzustellen. Als Katalysatoren werden basische Verbindungen, nämlich Alkalihydroxid bzw. Alkalialkoholat sowie Amine vorgeschlagen (DE-PS 1 002 342).

Nachteile dieses Verfahrens bestehen darin, daß die Alkalihydroxide bzw. Alkalialkoholate nach der Reaktion durch Neutralisieren zerstört werden müssen, während die Amine destillativ zurückgewonnen werden müssen, was häufig größeren Aufwand bedeutet. Weiterhin reagieren die Amine in erheblichem Ausmaß mit Acrylnitril zu Additionsprodukten, was Katalysatorverlust bedeutet.

Ziel der vorliegenden Erfindung ist es, diese Nachteile zu vermeiden.

Es wurde nun ein katalytisches Verfahren zur Herstellung von 5-Oxohexannitril aus Aceton und Acrylnitril bei erhöhter Temperatur gefunden, das dadurch gekennzeichnet ist, daß man als Katalysator ein Alkalifluorid oder ein Alkylammoniumfluorid, das sich von primären Aminen ableitet, verwendet.

Die Vorteile des neuen Verfahrens bestehen darin, daß der Katalysator weder mit Aceton noch mit Acrylnitril oder deren Reaktionsprodukt 5-Oxohexannitril in nennenswerter Weise zu Nebenprodukten reagiert und daher die oben genannten Katalysatorverluste vermieden werden. Weiterhin kann der Katalysator aufgrund seines ionischen Charakters aus dem organischen Reaktionsprodukt leicht wiedergewonnen werden, z.B. durch Auskristallisieren.

Als Katalysatoren verwendet man vorzugsweise Natrium- oder Kaliumfluorid oder Alkylammoniumfluoride, die sich von primären Alkylaminen ableiten, insbesondere solchen mit bis zu 6 C-Atomen. Besonders bevorzugt sind Kaliumfluorid, Ethylammoniumfluorid und Isopropylammoniumfluorid, vor allem aber Kaliumfluorid. Die Zusätze an Fluorid betragen im allgemeinen 0,01 bis 0,5 Mol, bevorzugt 0,05 bis 0,2 Mol, pro Mol eingesetztes Acrylnitril.

Die genannten Fluoride sind im allgemeinen bei Raumtemperatur in Aceton und Acrylnitril sowie im Reaktionsprodukt, das 5-Oxohexannitril enthält, schwer löslich. Die Fluoride müssen beim vorliegenden Verfahren jedoch nicht notwendigerweise vor der Umsetzung durch einen Zusatz von Lösemitteln in Lösung gebracht werden, sondern man kann sie supendieren, wobei sie auch nach der Umsetzung kristallin in der Reaktionslösung vorliegen und in üblicher Weise abgetrennt und zurückgewonnen werden können. Es ist jedoch vorteilhaft, die Fluoride vor der Umsetzung in einer solchen Menge Wasser zu lösen und dem Gemisch aus Aceton und Acrylnitril hinzuzufügen, daß sich ein zweiphasiges System aus organischer und wäßriger Phase bildet, das auch nach der Umsetzung erhalten bleibt und von dem die wäßrige Phase mit dem Katalysator in einfacher Weise abgetrennt und erneut zur Umsetzung verwendet werden kann. Die hierfür geeignete Wassermenge beträgt etwa 3 - 20 Gew.%, bezogen auf die Gewichtsmenge an eingesetztem Aceton und Acrylnitril. Der Zusatz von Wasser erleichtert somit - besonders bei der Durchführung des Verfahrens in kontinuierlicher Betriebsweise - nicht nur die Katalysatorrückgewinnung, sondern auch die Dosierung des Katalysators.

Das molare Verhältnis, in dem Aceton und Acrylnitril zum Einsatz kommen, beträgt im allgemeinen 1 : 1 bis 20 : 1, vorzugsweise 3 : 1 bis 8 : 1. Um die Polymerisation des Acrylnitrils während der Reaktion zu verhindern, ist es vorteilhaft, eine kleine Menge Polymerisationsinhibitor, wie Hydrochinon, zuzugeben.

Die günstigste Reaktionstemperatur beträgt im allgemeinen 100 bis 250°C, vorzugsweise 180 bis 230°C.

Der Druck hat geringen Einfluß auf die Reaktion, soll aber mindestens so hoch eingestellt werden - ggf. unter Zugabe von Inertgas - daß sich das Reaktionsgemisch in flüssigem Zustand befindet.

Beispiel 1

0,3 Mol Acrylnitril, 1,5 Mol Aceton und 0,1 g Hydrochinon werden mit 10 g einer wäßrigen Lösung, die 3 g Kaliumfluorid enthält in einem Autoklaven (Volumen 200 ml) während 2 Stunden auf 225°C erhitzt. Die organische Phase des zweiphasigen Reaktionsprodukts wird gaschromatographisch analysiert, sie enthält 0,075 Mol Acrylnitril und 0,14 Mol 5-Oxohexannitril. Der Umsatz an Acrylnitril beträgt 75 %; 5-Oxohexannitril ist mit einer Selektivität von 62 %, bezogen auf umgesetztes Acrylnitril entstanden. Die vom Reaktionsprodukt abgetrennte wäßrige Phase (8 g) wird zu einem Gemisch aus Aceton, Acrylnitril und Hydrochinon obiger Zusammensetzung gegeben und die Umsetzung wiederholt. Der Umsatz an Acrylnitril beträgt im zweiten Durchgang 71 %, die Selektivität zu 5-Oxohexannitril 61 %.

Beispiel 2

0,3 Mol Acrylnitril, 1,5 Mol Aceton und 0,1 g Hydrochinon werden mit 8 g einer wäßrigen Lösung, die 4 g Isopropylammoniumfluorid enthält, in einem Autoklaven (Volumen 200 ml) während 2 Stunden auf 225°C erhitzt. Die organische Phase des zweiphasigen Reaktionsprodukts wird gaschromatographisch analysiert, sie enthält 0,048 Mol Acrylnitril (Umsatz 84 %) und 0,165 Mol 5-Oxohexannitril (Selektivität 66 %).

Patentansprüche

1. Verfahren zur Herstellung von 5-Oxohexannitril aus Aceton und Acrylnitril bei erhöhter Temperatur, dadurch gekennzeichnet, daß man als Katalysator ein Alkalifluorid oder ein Alkylammoniumfluorid, das sich von primären Aminen ableitet, verwendet.

2. Verfahren nach Anspruch 1, dadurch gekennzeichnet, daß man als Katalysator Natriumfluorid, Kaliumfluorid oder ein Alkylammoniumfluorid verwendet, das sich von primären Alkylaminen mit bis zu 6 C-Atomen ableitet.

3. Verfahren nach Anspruch 1, dadurch gekennzeichnet, daß man als Katalysator Kaliumfluorid verwendet.

4. Verfahren nach einem der Ansprüche 1 bis 3, dadurch gekennzeichnet, daß man 0,01 bis 0,5 Mol des Fluorids, bevorzugt 0,05 bis 0,2 Mol, pro Mol Acrylnitril verwendet.

5. Verfahren nach einem der Ansprüche 1 bis 4, dadurch gekennzeichnet, daß man das Fluorid in Form einer wäßrigen Lösung einsetzt, wobei der Wasseranteil 3 bis 20 Gew. %, bezogen auf die Gewichtsmenge an Aceton und Acrylnitril, beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | C 07 C 121/34 |
| A | US-A-2 381 371 (SHANNON) | | C 01 D 3/02 |
| | | | |
| | --- | | |
| A | Chemical Abstracts Band 50, Nr. 13, 1956, Columbus, Ohio, USA, A.N. KOST et al. "Reduction with formic acid and its derivatives. III. Synthesis of substituted alpha-piperidones: A 24-hr. reaction of 26.5 g. $CH_2$:CHCN with 178 g. MeEtCO in the presence of EtONa in EtOH gave 27-30 % gamma-acetylva leronitrile", Spalte 2, Abstract Nr. 9410f & Zhur. Obshchei Khim. Band 25, 1955, Seiten 2464-2469 | | |
| | | | RECHERCHIERTE SACHGEBIETE (Int Cl. ³) |
| | --- | | |
| A | Journal of the Chemical Society - Chemical Communications", 1977, BELSKY "'Naked' Fluoride-catalysed Michael-Additions", Seite 237 | | C 01 D 3/02 |
| | | | C 07 C 121/34 |
| | --- | | |
| A | Angewandte Chemie, Band 93, Heft 9, September 1981, MATSUMOTO "Fluoride-katalysierte Michael-Addition bei hohen Drücken", Seiten 803-804 | | |
| | ----- | | |

Der vorliegende Recherchenbericht wurde für alle Patentansprüche erstellt.

| Recherchenort **BERLIN** | Abschlußdatum der Recherche **17-01-1983** | Prüfer **BREW C.H.** |
|---|---|---|